# EUROPEAN PATENT APPLICATION

(11) **EP 1 882 736 A1**
(43) Date of publication of application: **30.01.2008**
(21) Application number: 06746283.8
(22) Date of filing: 11.05.2006
(51) Int. Cl.: C12M 3/00

(54) **CELL CULTURE CONTAINER**

(30) Priority: 17.05.2005 JP 2005144759
(71) Applicant: KURARAY CO., LTD., Kurashiki-shi, Okayama 710-8622 (JP)
(72) Inventor: TAZAKI, Go, Tsukuba-shi, Ibaraki 3050841 (JP); NISHI, Taiji, Okayama 710-8691 (JP); FUKUDA, Motohiro, Tsukuba-shi, Ibaraki 3050841 (JP); KIRITA, Yasuzo, Kurashiki-shi, Okayama 7108622 (JP)
(74) Representative: Vossius & Partner
(86) International application number: PCT/JP2006/309474
(87) International publication number: WO 2006/123570

(57) **Abstract**

It is intended to provide a cell culture chamber in a shape suitable for cell culture. It is also intended to provide a cell culture chamber which is inexpensive and facilitates observation. A cell culture chamber (100) includes a cover (10), a chamber body (20) and a plate-shaped base (30). The cover (10) covers an opening (21) of the chamber body (20). The chamber body (20) contains a culture such as cells. The plate base (30) covers a through hole (24) formed at the bottom (23) of the chamber body (20). Moreover, the plate base (30) has fine pits and projections formed thereon for culturing the culture.

## Description

### Technical Field

The present invention relates to a cell culture chamber that is used for a bioassay using cultured cells in evaluating the effect of a drug or the like and assessing its toxicity and for a cell culture process intended to provide treatment.

### Background Art

A technique of using a cell that is isolated from tissue for assessment and examination is essential in biotechnology-related fields. Such a technique is widely used for diagnosis of diseases and pathoses, search for a new drug and evaluation of its effect, animal inspection, plant inspection, assessment of an environment pollutant and so on.
Although an isolated cell is immediately used for assessment in some cases, it is cultured in a culture dish or a test tube by a cell culture process in most cases. Various examinations are conducted in such a cell culture system.

An assay normally uses a uniform culture system and evaluates the effect of a drug or the like by changing its amount, concentration and so on. Thus, a culture vessel to be used for cell culture is the one that is formed uniformly. A culture dish is generally used as a culture vessel. Generally used culture dishes are: a petri dish, a 6-well plate, and 12-well, 48-well and 96-well plates (cf. Patent document 1). Given the recent trend for micro-samples, a 384-well plate that is composed of a larger number of culture dishes with a smaller diameter is increasingly used nowadays.
Further, a culture chamber with a grid coordinate for locating a specific portion of a culture is proposed (cf. Patent document 2). The bottom of such a culture dish and a culture chamber is flat-plate shaped.

However, if tissue cell culture is performed in a cell culture dish or culture chamber having a flat plate bottom, cells are thinly elongated into a form without orientation and thus cease to exhibit a function which they have in vivo. As a way to check the activity of cells, a change in PH due to discharge of waste products and release of carbon dioxide may be measured using an electrochemical sensor or the like. Although there is an attempt to compare measured data of biological tissue with measured data of cells cultured in a culture dish, a value that indicates data of biological tissue cannot be reproduced in a culture dish with the current state of the art. A possible reason is that, although one well plate is chamber-shaped, it is as if culture on a flat plate for cells with a size of several microns to several tens of microns. Particularly, in the growth of tissue cells which are difficult to culture, such as hepatocytes, it is further difficult to perform the function they have in vivo.

As an approach to overcome the above problem, a technique of forming a fine chamber pattern that is suitable for the growth of tissue cells on a culture dish and culturing cells in the fine chamber pattern is proposed (cf. Patent document 3). This technique assists the development of the function which cells have in vivo by culturing cells in the fine chamber pattern so as to grow the cells three dimensionally.

However, this technique is only applicable to limited bioassays or cell culture designed to limited treatment with the current state of the art. For example, myocardial cells in the biological heart beat in response to the transmission of an electric signal from the brain. For the beating function, the biological myocardial cells are composed of an arrangement with orientation. Although the studies related to tissue regeneration of artificial organs in the biotechnology field thus require cell culture with control of the extension direction just like biological tissue, the existing culture in a culture dish has difficulty in the three dimensional culture and fails to control the extension direction, thus not applicable to the purposes of research assessment.

Further, as one of myocardial cell culture designed to treatment, there is a study of grafting cultured myocardial tissue to a part of myocardial tissue that is necrotic due to myocardial infarction in order to save life. Normally, the whole heart beats greatly by an electric signal from the brain. When a part of myocardial tissue becomes necrotic due to myocardial infarction, the signal transmission within myocardium is blocked, and the heart repeats small contraction called fibrillation. As a result, thrombus is generated due to blood retention in the heart, and if it is delivered to brain tissue, it gives rise to the secondary case such as cerebral infarction. If the fibrillation persists for a long time, it can lead to death. Because the treatment of such a case aims not at the completion of an artificial organ itself but at the substitution of partial tissue, early implementation is desired.

However, because the existing culture in a culture dish has difficulty in the three dimensional culture and fails to control the extension direction, it is not applicable to such a purpose as well.

[Patent document 1] Japanese Unexamined Patent Application Publication No. 11-169166
[Patent document 2] Japanese Unexamined Patent Application Publication No. 2001-17157
[Patent document 3] Japanese Unexamined Patent Application Publication No. 8-322593

### Disclosure of the Invention

### Problems to be solved by the Invention

As described above, if cell culture is performed in a conventional cell culture chamber, a cell is thinly elongated and ceases to exhibit a function which it has in vivo.
The present invention has been accomplished to solve the above problems and a first object of the present invention is to provide a cell culture chamber having a shape suitable for cell culture. Further, a second object of the present invention is to provide a cell culture chamber that allows easy observation at low cost.

### Means for solving the problems

To these ends, according to one aspect of the present invention, there is provided a cell culture chamber that at least includes a chamber body to contain a culture, a cover to cover an opening of the chamber body, and a plate base to cover a through hole at a bottom portion of the chamber body, wherein the plate base has fine pits and projections for cultivating the culture.

According to another aspect of the present invention, there is provided a cell culture chamber that includes a chamber body to contain a culture, and a cover to cover an opening of the chamber body, wherein a bottom portion of the chamber body has fine pits and projections for cultivating the culture.

A thickness of the plate base is preferably smaller than a thickness of the bottom portion of the chamber body. Particularly, the thickness of the plate base is preferably 0.03 mm to 1 mm.

It is also preferred that a space structure which is formed by the fine pits and projections and in which a culture is placed and cultivated has a width of 0.01 µm to 1000 µm, a length of 0.01 µm to 1000 µm, and a height of 0.01 µm to 1000 µm. It is further preferred that a plurality of space structures are formed by the fine pits and projections, and each space structure is connected with at least one adjacent space structure.

Preferably, surface treatment is performed in a region having the fine pits and projections. The plate base is preferably a transparent resin base. An area of the plate base is preferably 10% to 60% of an area of the bottom portion of the chamber body.

It is preferred that the bottom portion of the chamber body has a recessed portion in a region including the through hole, and the plate base is inserted into the recessed portion. Particularly, it is preferred that the plate base is detachably fixed to the recessed portion by a fitting member. The fitting member is preferably made of a self-adhesive resin. A thickness of the fitting member is preferably equal to or larger than a depth of the recessed portion.

The through hole at the bottom portion is preferably circular or rectangular. A shape of the plate base is also preferably circular or rectangular. Further, it is preferable that the cover or the chamber body has an opening for perfusion.

### Advantages of the Invention

The present invention can provide a cell culture chamber having a shape suitable for cell culture and further provide a cell culture chamber that allows easy observation at low cost.

### Brief Description of the Drawings

Fig. 1 is a partial sectional view of a cell culture chamber according to the present invention;
Fig. 2 is a partial sectional view of a cover of a cell culture chamber according to the present invention;
Fig. 3 is a partial sectional view of a chamber body of a cell culture chamber according to the present invention;
Fig. 4 is an enlarged partial sectional view of a cell culture chamber according to the present invention;
Fig. 5 is an enlarged partial sectional view of a cell culture chamber according to the present invention;
Fig. 6A is an enlarged partial sectional view of a cell culture chamber according to the present invention;
Fig. 6B is an enlarged partial sectional view of a cell culture chamber according to the present invention;
Fig. 7 is an enlarged partial sectional view of a cell culture chamber according to the present invention;
Fig. 8 is a top view showing fine pits and projections formed on a plate base of a cell culture chamber according to the present invention;
Fig. 9 is a top view showing fine pits and projections formed on a plate base of a cell culture chamber according to the present invention;
Fig. 10 is a top view showing fine pits and projections formed on a plate base of a cell culture chamber according to the present invention;
Fig. 11A is a top view showing fine pits and projections formed on a plate base of a cell culture chamber according to the present invention;
Fig. 11B is a sectional view showing fine pits and projections formed on a plate base of a cell culture chamber according to the present invention;
Fig. 12A is a top view showing fine pits and projections formed on a plate base of a cell culture chamber according to the present invention;
Fig. 12B is an enlarged partial top view showing fine pits and projections formed on a plate base of a cell culture chamber according to the present invention;
Fig. 12C is an enlarged partial sectional view showing fine pits and projections formed on a plate base of a cell culture chamber according to the present invention;
Fig. 13 is a perspective view showing fine pits and projections formed on a plate base of a cell culture chamber according to the present invention;
Fig. 14 is a perspective view showing fine pits and projections formed on a plate base of a cell culture chamber according to the present invention;
Fig. 15 is a perspective view showing fine pits and projections formed on a plate base of a cell culture chamber according to the present invention;
Fig. 16 is a perspective view showing fine pits and projections formed on a plate base of a cell culture chamber according to the present invention;
Fig. 17 is a perspective view showing fine pits and projections formed on a plate base of a cell culture chamber according to the present invention;
Fig. 18 is a perspective view showing fine pits and projections formed on a plate base of a cell culture chamber according to the present invention; and
Fig. 19 is a perspective view showing fine pits and projections formed on a plate base of a cell culture chamber according to the present invention.

### Description of Reference Numerals

- 10: cover
- 20: chamber body
- 30: plate base
- 40: fitting member
- 100: cell culture chamber

### Best Modes for Carrying Out the Invention

If culture of tissue cells is performed in a commercially available cell culture dish (petri dish or well plate), the tissue cells are thinly elongated into a form without orientation. Although researchers attempt to compare measured data of biological tissue with measured data of cells cultured in a culture dish about a change in PH due to discharge of waste products and release of carbon dioxide using an electrochemical sensor or the like as a way to check the activity of cells, a value that indicates data of biological tissue cannot be reproduced in a culture dish with the current state of the art. It is therefore determined that cells which are cultured in a commercially available cell culture dish do not exhibit the function which they have in vivo. In view of the foregoing, a study has started for promoting the development of the function which cells have in vivo by forming a fine chamber pattern that is suitable for the growth of tissue cells on a culture dish and culturing cells in the fine chamber pattern so as to grow the cells three dimensionally.

However, in considering the application of the tissue that is composed of the cells cultured in the culture dish having the fine chamber pattern to actual treatment, because the tissue that is composed of the cultured cells does not have the same sequence structure as in a living body, there is still a large hurdle to overcome to enable the application to treatment even if the three dimensional culture of cells becomes possible. For example, in the application to the case where the signal transmission within myocardium is blocked and the heart enters fibrillation as a result that a part of myocardial tissue becomes necrotic due to myocardial infarction, it is impossible to restore the signal transmission of myocardium and reproduce normal heart beating without grafting the tissue in which the extension direction of myocardial cells is controlled.

A cell culture method of the present invention enables not only the three-dimensional growth of cultured cells but also the control of the extension direction of cultured cells by forming fine pits and projections, and also enables the obtainment of tissue that is similar to biological tissue with a desired area and thickness by coupling the cells cultured in several space structures. It is thereby possible to realize the cultured tissue that is desired by doctors, medical engineering researchers and patients. The fine pits and projections may be implemented by forming a plurality of side walls and a plurality of space structures which are made by the side walls and in which cultured cells are placed, and creating openings in the side walls to form a coupling structure in which a communication is established among the plurality of space structures.
The plurality of space structures are created by forming the plurality of side walls, and the area size of the space structures is set according to an intended usage.

In cell culture, a desmosome to serve as pseudo-scaffolding is formed on a side wall surface and/or a space structure surface. Cells grow three-dimensionally at the center in the space structure and form a framework structure. A cell that extends to the opposite side wall is coupled with a cell that is cultured in a different space structure through an opening, thus enabling the production of cultured tissue in which the extension direction is controlled. It is assumed that the control of the extension direction is made possible in a variety of culture systems by setting the dimensions of a side wall, a space structure and an opening according to cell species to be cultured. The opening is a structure through which space structures that are formed by side walls are connected with each other. For example, a space between side walls, a pit structure of a side wall, a tunnel structure formed inside a side wall and so on are effective as an opening to couple desmosomes of cells.

The dimensions of a plate base, a side wall that is formed at the bottom of a cell culture chamber and a space structure that is formed by side walls should be within the range that is most suitable for the purpose of cell culture. If the space structure that is formed by side walls is too large, cells are thinly elongated to lose a three-dimensional structure, so that its extension direction is uncontrollable as in the culture on a flat plate. If, on the other hand, the space structure is too small, cells fail to enter the space structure. Accordingly, the dimensions of the space structure are preferably in the range to contain one or a plurality of cells according to cell species to be cultured.

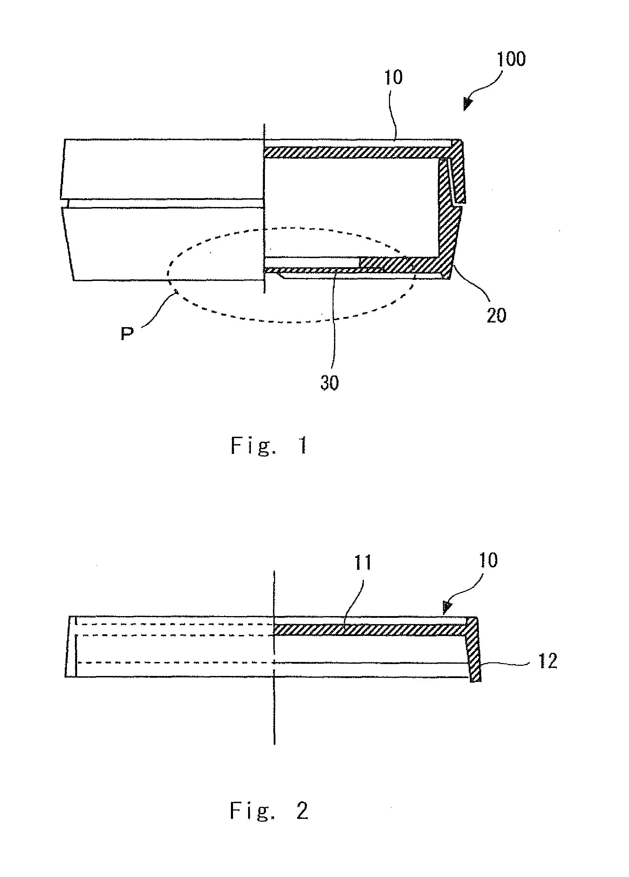

The structure of a cell culture chamber according to the present invention is described hereinafter with reference to the drawings. Fig. 1 is a partial sectional view showing a cell culture chamber with a cover attached thereto. A cell culture chamber 100 of the present invention includes a cover 10, a chamber body 20, and a plate base 30. The cell culture chamber 100 to which the cover 10 is attached has a column shape with a top-face and bottom-face diameter of 20 mm to 80 mm and a height of 5 mm to 40 mm, for example.

As shown in the partial sectional view of Fig. 2, the cover 10 has a top face portion 11 and a side wall portion 12. The top face portion 11 of this embodiment is composed of a circular flat plate. The side wall portion 12 is formed to project substantially perpendicularly from the edge of the top face portion 11 in a cylindrical shape. In Fig. 2, the side wall portion 12 is formed to have an opening on the downside. Although the cover 10 of this embodiment is cylindrical shaped, it may be square shell shaped. To the opening that is formed by the side wall portion 12, a side wall portion 22 of the chamber body 20, which is described later, is inserted. Because the height of the side wall portion 12 of the cover 10 is lower than the height of the side wall portion 22 of the chamber body 20 in this embodiment, the end of the side wall portion 12 of the cover 10 is located at about the middle of the side wall portion 22 of the chamber body 20 in the fitted state as shown in Fig. 1.

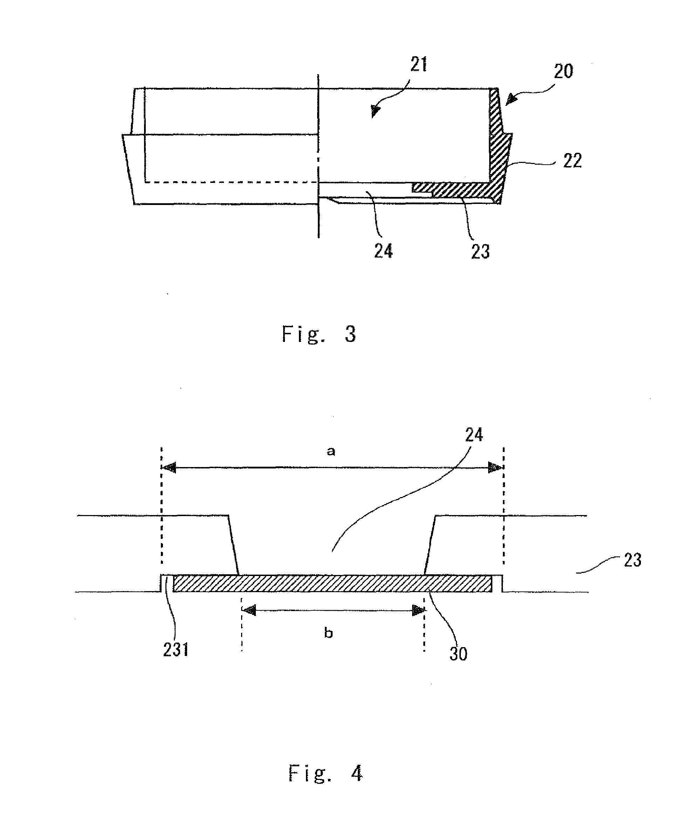

As shown in Fig. 3, the chamber body 20 includes the side wall portion 22 and a bottom portion 23, by which an opening 21 that opens upward is formed. A culture and a culture solution are contained in the opening 21 of the chamber body 20. The opening 21 is covered and hermetically sealed when the cover 10 is attached. The bottom portion 23 of this embodiment has a through hole 24 at its center, and it is a ring-shaped flat plate. Although the through hole 24 of this embodiment is circular, it may be polygonal such as square. The side wall portion 22 is formed to project substantially perpendicularly from the edge of the bottom portion 23 in a cylindrical shape. In Fig. 3, the side wall portion 22 is formed to have an opening 21 on the upside.

The side wall portion 22 of the chamber body 20 may have a through hole (opening) to allow perfusion. Although both of an inlet and an outlet of the through hole for perfusion may be formed in the side wall portion 22 of the chamber body 20, an inlet may be formed in the top face portion 11 of the cover 10.
The cover 10 and the chamber body 20 are made of a plastic or glass material, for example. Because a high accuracy is not required for the cover 10 and the chamber body 20, it is preferred to use a lower-cost material than the plate base 30.

The plate base 30 is a separate unit from the chamber body 20. It is thus possible to select a different material from the cover 10 and the chamber body 20 as a material of the plate base 30, which requires fine patterning. For example, with the use of a low-cost material for the cover 10 and the chamber body 20 and a high-cost material for the plate base 30 which requires fine patterning, the cell culture chamber as a whole can be manufactured at low cost with high accuracy.

Fine pits and projections are formed on one surface of the plate base 30. A culture is placed and cultivated on the surface of the plate base 30 having the fine pits and projections. The fine pits and projections are formed to cultivate a culture and, more specifically, to perform control such as adhesion, growth, differentiation, extension and orientation on a culture. Particularly, because the plate base 30 is a flat plate, the fine pits and projections can be formed easily compared with the case of forming the fine pits and projections at the bottom of a cell culture chamber. A specific structure of the fine pits and projections is described in detail later.

The plate base 30 has a size enough to cover the through hole 24 that is formed in the bottom portion 23 of the chamber body 20. Because the through hole 24 is circular, the plate base 30 of this embodiment is a circular flat plate with a larger diameter than the through hole 24. The shape of the plate base 30 is not necessarily circular, and it may be polygonal such as square. The area of the plate base 30 is preferably about 20% to 60%, and more preferably about 40% to 60%, of the area of the bottom portion 23 in terms of preventing breakdown of the plate base 30. If it is less than 20%, the area to cultivate a culture is too small, and if it is more than 60%, the adhesion of the flat-plate base is difficult.

Fig. 4 is a sectional view showing the portion P of Fig. 1 in an enlarged scale. As shown in Fig. 4, a recessed portion 231, to which the plate base 30 is inserted, is formed in the vicinity of the through hole 24 at the bottom portion 23 of the chamber body 20. Thus, the thickness of the bottom portion 23 is thinner in the vicinity of the through hole 24 than in the other part. The recessed portion 231 has a shape of a circular notch. A diameter a of the recessed portion 231 is 10 mm to 50 mm, and a diameter b of the through hole 24 is 5 mm to 40 mm, for example. The plate member 30 is bonded and fixed to the recessed portion 231 by an adhesion bond, for example. The presence of the recessed portion 231 at the bottom portion 23 facilitates the positioning when adhering the plate base 30 to the chamber body 20, thereby preventing the seepage of an adhesion bond or the like during the cultivation of a culture.

The thickness of the plate base 30 is preferably 0.03 mm to 1 mm and, more preferably, 0.1 mm to 0.3 mm, for example. If the thickness is less than 0.03 mm, it is difficult to process the fine pits and projections. If the thickness exceeds 1 mm, it is unable to obtain a sufficient focal length in the microscope observation. Particularly, when observing a culture with the use of a microscope or the like at high magnification, it is preferred to select a thickness according to the magnification. Especially, when observing a culture with an objective lens at high magnification of 100x or above, the thickness is preferably 0.2 mm or less.

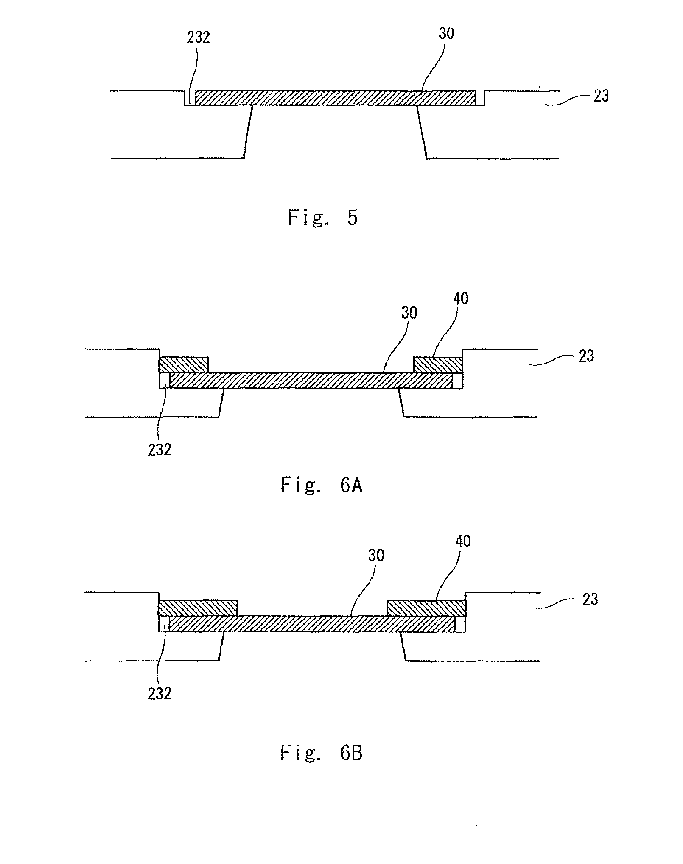

As shown in Fig. 5, a recessed portion 232, to which the plate base 30 is inserted, may be placed inside. In such a case, the plate base 30 is inserted into the recessed portion 232 from the inner side. The thickness of the plate base 30 is set to be equal to or larger than the depth of the recessed portion 232, thereby suppressing the effect of retention of a culture solution during perfusion.

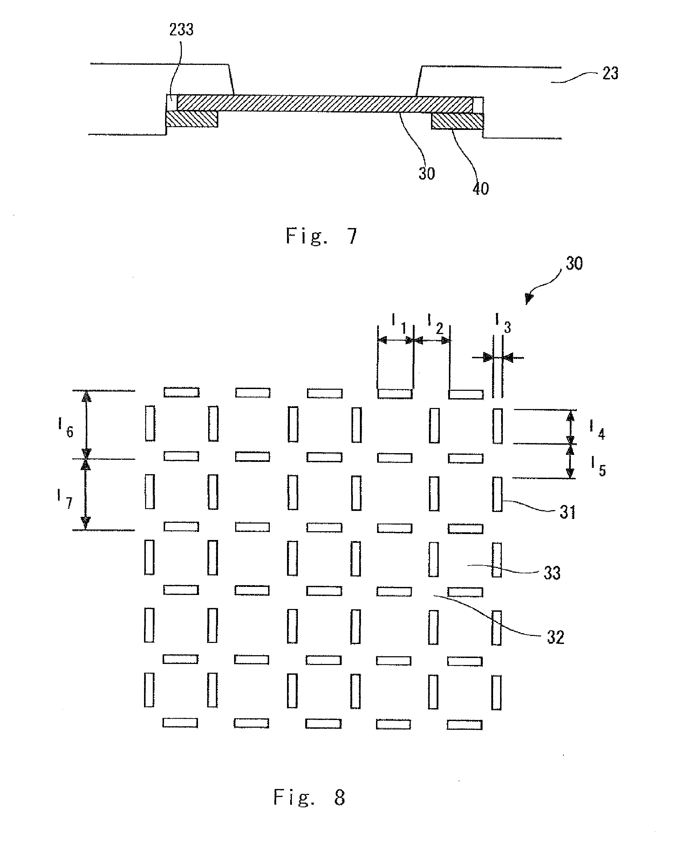

Further, as shown in Fig. 6A, a fitting member 40 for pressing and fixing the plate base 30 which is inserted into the recessed portion 232 that is formed on the inside may be placed. As shown in Fig. 7, the fitting member 40 may be used also when pressing and fixing the plate base 30 to the recessed portion 233 that is formed on the outside.
Furthermore, if the inside diameter of the fitting member 40 is set smaller than the inside diameter of the through hole in the recessed portion 23 as shown in Fig. 6A, it is possible to detach the plate base 30 easily without causing a damage to the fine pattern region where a culture is cultivated when removing the plate base 30 by pressing the plate base 30 from the opposite side of the fitting member 40.

The fitting member 40 of this embodiment has a ring shape having a through hole at the center, and it may be made of a commercially available sealing material. The fitting member 40 is made of a silicone rubber resin having self-bonding properties, such as polydimethylsiloxane, for example.

With the use of the structure that fixes the plate base 30 by the fitting member 40, it is possible to remove only the plate base 30 after cultivating a culture and perform grafting or the like.

It is preferred to perform surface treatment on the plate base 30 in order to remove bubbles and immobilize cells. It is effective to make the base surface hydrophilic in order to remove bubbles and bring a culture solution in contact with the base surface. Various techniques may be used as a method of the surface treatment for giving the hydrophilic properties to the base surface. For example, there are a technique of using low temperature plasma treatment, corona discharge treatment, ultraviolet irradiation and so on, a technique of dissolving a hydrophilic polymeric material in water solution and coating it, vapor deposition polymerization, plasma polymerization and so on. Further, as a method for enhancing the solvent resistance of the coated polymeric material in a culture solution, a technique of grafting a coating material with a functional group on the base surface is known.

In order to immobilize cells, there is a technique of making the base surface hydrophobic, depositing an inert metal, or applying collagen or the like which is a protein to enhance the adhesion of cells. As a method to give the hydrophobic properties to the base surface, there is a technique of deposition of a hydrophobic metal by sputtering, vapor deposition or the like, vapor deposition polymerization, deposition of a polymeric material by plasma, and so on. An inert metal may be deposited by vapor deposition or sputtering of a metal, for example. It is possible to coat some portion during the surface treatment so as to modify the properties of a given portion.

A material that is used for the plate base 30 is preferably plastic in terms of the efficiency in the surface treatment. The plate base 30 may be a glass base on which fine pits and projections are formed by 2P resin. Further, a transparent material that allows transmitted light observation is preferred when observing the growth process of cells with the use of a fluorescence microscope, for example. Although a resin material is not particularly limited, acrylic resin, styrene resin, acrylic-styrene copolymer resin (MS resin), polycarbonate resin, ester resin such as polyethylene terephthalate or polyglycolic acid, vinyl acetate resin such as polyvinyl alcohol or ethylene-vinyl alcohol copolymer, thermoplastic elastomer such as styrene elastomer, vinyl chloride resin, silicone resin such as polydimethylsiloxane, polyvinyl butyral resin, olefin resin such as polyethylene or cycloolefin and so on may be used, for example. Such a resin may be a copolymer of a monomer.

In the case of forming the cell culture chamber 100 and the plate base 30 using a plastic material, it is preferred to use a molding method that forms a resin molded part using a metal structure as a mold. For the cell culture chamber 100, the metal structure may be produced by machining a steel material using a ball end mill, for example. For the plate base 30, it may be produced by forming a resist pattern by photolithography using UV light as an exposure light source, depositing nickel on the surface by sputtering, and then performing electroplating.

Although a technique of forming the resin molded part is not particularly limited, injection molding, press molding, monomer casting, solution casting, or roll transfer by extrusion molding may be used, for example. The use of the injection molding is preferred for its high productivity and transcription property. It is preferred to use the injection molding for the cover 10 and the chamber body 20 and use the press molding for the plate base 30, which requires high accuracy. When forming a resin molded part by the injection molding using a metal structure with a given size as a mold, it is possible to reproduce the shape of a metal structure on a resin molded part at a high reproduction rate. The transcription rate may be checked by using an optical microscope, a scanning electron microscope (SEM), a transmission electron microscope (TEM) and so on.

Such a resin may contain one or more than one agent of lubricant, light stabilizer, heat stabilizer, antifogging agent, pigment, flame retardant, antistatic agent, mold release agent, antiblocking agent, ultraviolet absorbent, antioxidant and so on according to need.

An exemplary structure of the fine pits and projections that are formed on the plate base 30 is described hereinafter with reference to Fig. 8. Fig. 8 is a top view of the plate base 30. As shown therein, a plurality of side walls 31 are arranged in lattice fashion. An opening 32 is made between one side wall 31 and an adjacent side wall 31. A region that is surrounded by four side walls 31 is a space structure 33. In this embodiment, all of the space structures 33 have the same area.

In the space structure 33 to culture cells, a wall portion of the side wall 31 or a bottom portion, of the space structure 33 may have finer pits and projections for promoting the growth of cells. The finer pits and projections facilitate the formation of a desmosome called pseudo-scaffolding which is required for the immobilization of cells, thereby promoting the differentiation and the growth of cells.

The width of the space structure 33 is preferably 0.01 µm to 1000 µm, and more preferably 5 µm to 500 µm. The length of the space structure 33 is preferably 0.01 µm to 1000 µm, and more preferably 5 µm to 500 µm. The height of the space structure 33 is preferably 0.01 µm to 1000 µm, and more preferably 5 µm to 500 µm. The width of the side wall 31 is preferably 0.01 µm to 1000 µm, and more preferably 1 µm to 500 µm. The length of the side wall 31 is preferably 0.01 µm to 1000 µm, and more preferably 1 µm to 500 µm. The height of the side wall 31 is preferably 0.01 µm to 1000 µm, and more preferably 1 µm to 500 µm.

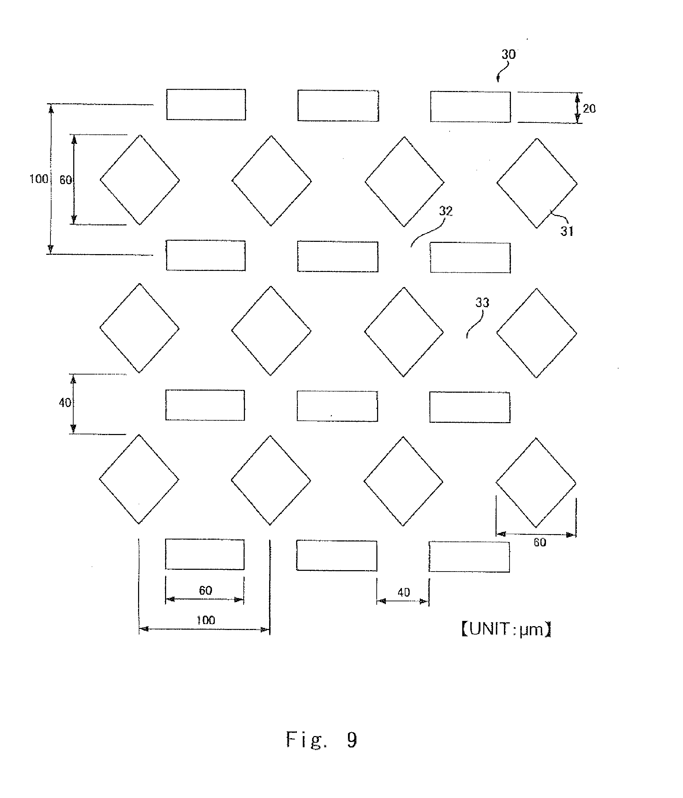

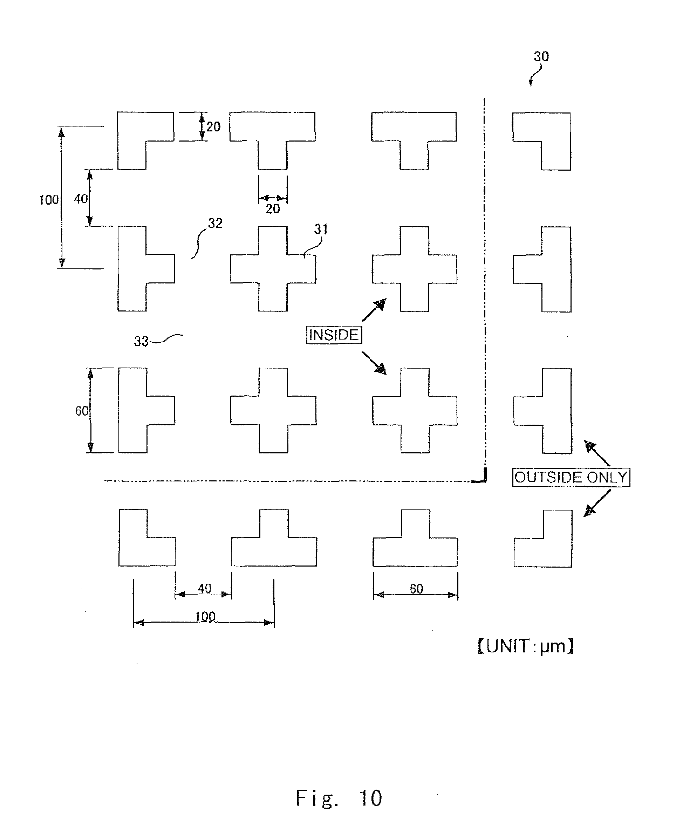

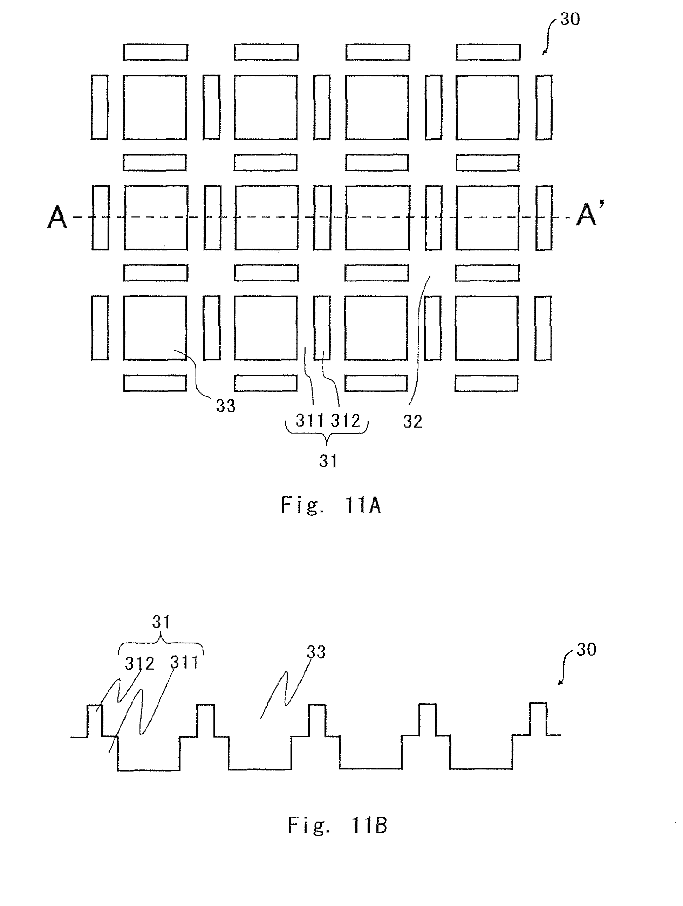

In this embodiment, the height of the side wall 31 is 20 µm, the width (13) is 10 µm, and the length (11, 14) is 60 µm. A distance (12, 15) between adjacent side walls 31 is 40 µm, and a distance (16, 17) between adjacent parallel side walls 31 is 100 µm. The size of the fine pits and projections is not limited to the above example, and it may be selected appropriately according to the kind of a culture or the like. Further, the fine pits and projections are not limited to the above-described shape, and they may have the shapes as shown in Figs. 9 and 10. Further, they may have the shapes as shown in Figs. 11A to 18, although not restricted thereto.

In the shape shown in Figs. 11A and 11B, the pit and projection pattern is formed like a two-level, step. Fig. 11A is a plan view, and Fig. 11B is a sectional view along line A-A' in Fig. 11A. A two-level side wall 31 is formed on a culture surface of the plate base 30. Specifically, rectangular second side walls 312 are formed in matrix on first side walls 311 that are arranged in lattice fashion. A space that is created by the first side walls 311 and the second side walls 312 serves as the space structure 33. Thus, a space between the adjacent first side walls 311 and a space between the adjacent second side walls 312 form the space structure 33. Specifically, the space structure 33 for culturing cells is formed by the space between one first side wall 311 and an adjacent first side wall 311 and between one second side wall 312 and an adjacent second side wall 312. The first side wall 311 and the second side wall 312 are formed substantially perpendicular to the bottom surface. Accordingly, the space structure 33 has a step-like shape with two different levels. As shown in Fig. 11A, the first side wall 311 is arranged in lattice fashion so as to surround the four sides of the rectangular space structure 33. The second side wall 312 is arranged in island shape on the first side wall 311 between the adjacent space structures 33. The second side wall 312 is formed on each of the four sides of the rectangular space structure 33.

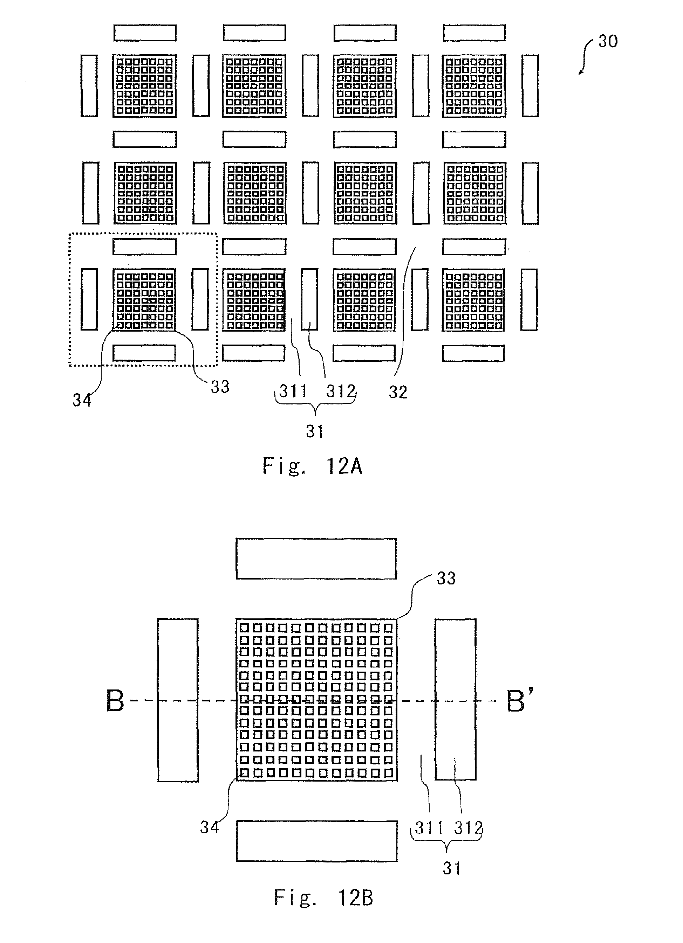

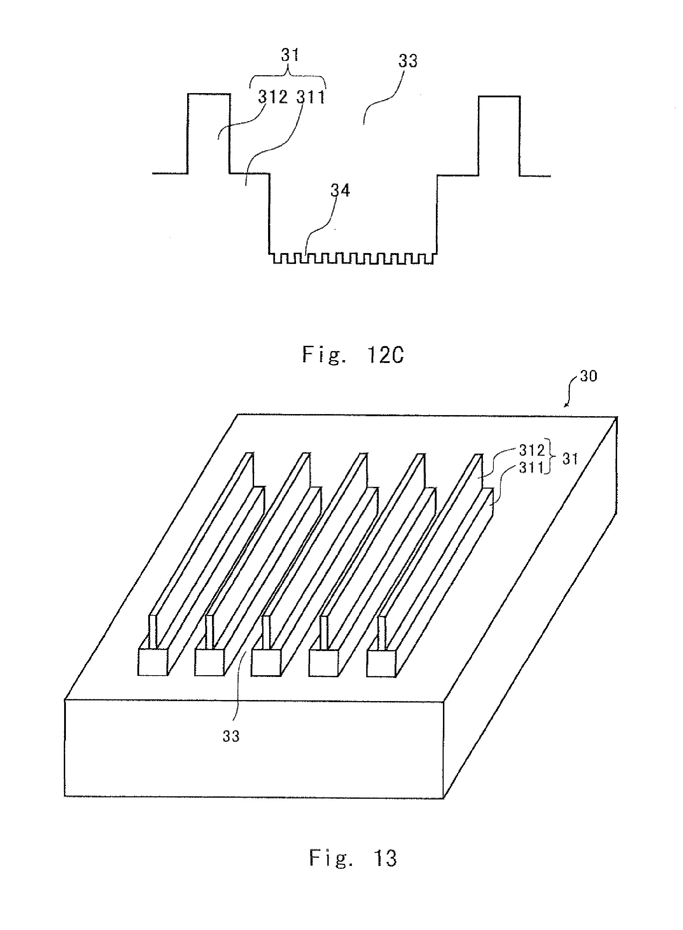

In the shape shown in Figs. 12A to 12C, a pit 34 is further formed on the bottom surface of the space structure 33 which is shown in Figs. 11A and 11B. Fig. 12A is a plan view, Fig. 12B is a plan view showing the structure within the dotted line in Fig. 12A in an enlarged scale, and Fig. 12C is a sectional view along line B-B' in Fig. 12B. The pit 34 is formed on the bottom surface between the adjacent first side walls 311. A plurality of pits 34 are formed between the adjacent first side walls 311. Accordingly, the cross-section of the plate base 30 has a step-like shape with three different levels in the vicinity of the first side walls 311. The pits 34 are arranged in matrix on the bottom surface of the space structure 33.

In the shape shown in the perspective view of Fig. 13, two-level step-like side walls 31 are arranged in a row. Each side wall 31 lies along the depth direction. The space structure 33 between the adjacent side walls 31 is a recessed groove through which a culture solution flows. The adjacent space structures 33 are connected with each other outside the side wall 31. The space structure 33 between the adjacent side walls 31 serves as a culture medium for culturing cells. With the shape shown in Fig. 13, a significant increase in the culture medium is attained. On the surface of the plate base 30, the direction in which the plurality of side walls 31 are arranged is a width direction, and the direction orthogonal to the width direction is a depth direction. The first side wall 311 and the second side wall 312 that is formed on the first side wall 311 have substantially the same size in the depth direction.

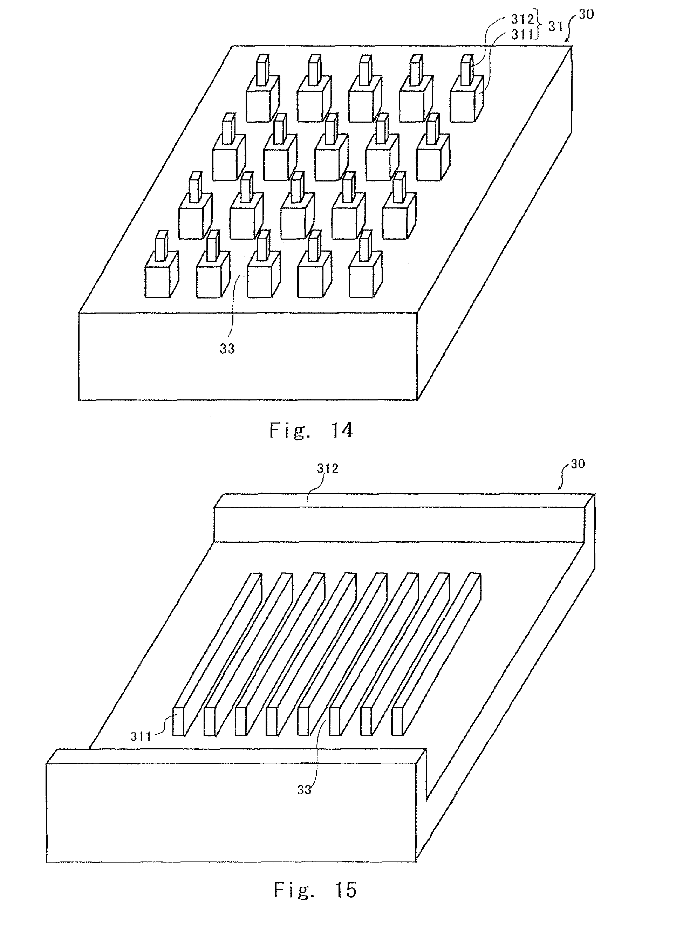

In the shape shown in the perspective view of Fig. 14, two-level step-like side walls 31 are arranged in matrix. Specifically, in the embodiment of Fig. 14, the second side wall 312 which is smaller than the first side wall 311 is placed on the first side wall 311. The side walls 31, each composed of the first side wall 311 and the second side wall 312, are arranged in matrix. The adjacent space structures 33 are connected with each other above each first side wall 311. Cells are cultured in the space structure 33 between the side walls 31. Thus, the space structure 33 serves as a culture medium of cells. With the shape shown in Fig. 14, a significant increase in the culture medium is attained.

In the shape shown in the perspective view of Fig. 15, the first side wall 311 and the second side wall 312 which is higher than the first side wall 311 are placed in different locations. Thus, the first side wall 311 and the second side wall 312 having different heights are placed separately. Specifically, the second side wall 312 is placed at each of both ends of the plate base 30, and the plurality of first side walls 311 are placed between the second side walls 312. The second side wall 312 is placed along the end edge of the plate base 30. The second side wall 312 is higher than the first side wall 311 that is placed in the center. Accordingly, the second side wall 312 serves as a bridge that is higher than a side wall 16 of the first side wall 311. This enables the retention of PH and the prevention of diffusion of output substances from cells. The first side walls 311 are arranged in a row along the longitudinal direction of the second side wall 312. Each first side wall 311 is placed in the direction orthogonal to the longitudinal direction of the second side wall 312. Each of the side walls lies along the depth direction. The space structure 33 between the adjacent side walls serves as a recessed groove through which a culture solution flows. A culture solution thereby flows in the direction orthogonal to the second side wall 312. In other words, a culture solution flows from one end to the other end via the center part of the plate base 30. This allows the prevention of diffusion of output substances. Further, the adjacent space structures 33 are connected with each other above each first side wall 311. This allows circulation of a culture solution.

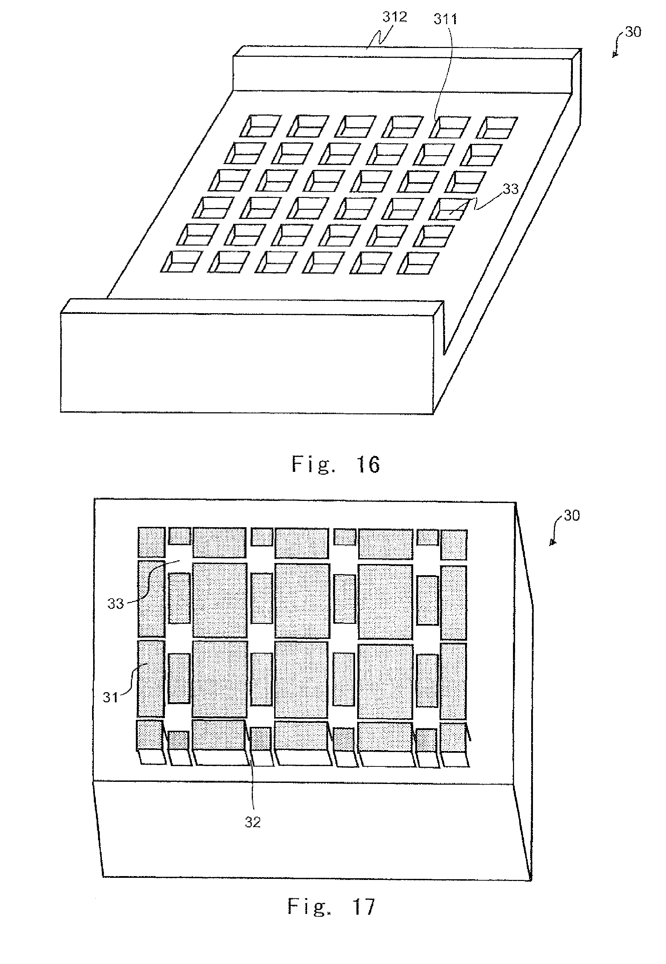

In the shape shown in the perspective view of Fig. 16, the space structures 33 that are formed by the first side walls 311 as in the embodiment shown in Fig. 15 are formed as a plurality of pits on the bottom surface of the plate base 30. The second side wall 312 is placed at each of both ends of the plate base 30. Thus, the two second side walls 312 are placed along the end sides of the plate base 30.

In the shape shown in the perspective view of Fig. 17, a plurality of space structures 33 are formed on one base. The space structures 33 are arranged vertically and horizontally on the base surface. A groove-shaped opening 32 is formed between the space structures 33. On the surface of the plate base 30, the horizontal direction in Fig. 17 is a width direction, and the vertical direction orthogonal to the width direction is a depth direction. The embodiment of Fig. 17 shows the plate base 30 having four space structures 33 in the width direction and three space structures 33 in the depth direction. Thus, twelve space structures 33 are formed in matrix on the plate base 30. The space structures 33 that are adjacent in the width direction are connected through one opening 32. On the other hand, the space structures 33 that are adjacent in the depth direction are connected through two openings 32. Specifically, the first side wall 31 is formed in the opening 32 between the space structures 33 adjacent in the depth direction. The first side wall 31 sectionalizes the opening 32 between the space structures 33 that are adjacent in the depth direction into two parts.

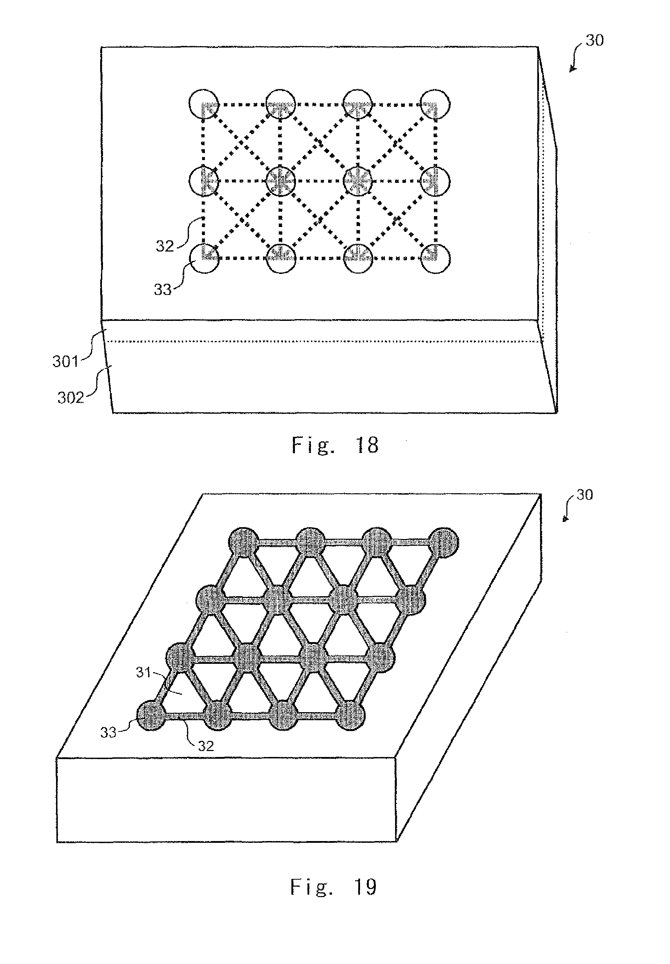

In the shape shown in the perspective view of Fig. 18, the plate base 30 is formed by adhering two bases together. Specifically, the plate base 30 is composed of a first base 301 and a second base 302 that are adhered with each other. The first base 301 has a through hole to serve as the space structure 33. Thus, a joint surface of the second base 302 serves as the bottom surface of the space structure 33. A fine groove to form the opening 32 is formed on the surface of the second base 302. Then, the first base 301 having the through hole and the second base 302 having the opening 32 are closely contacted with each other. The surface of the second base 302 on which the opening 32 is formed serves as a joint surface to be adhered. The opening 32 of the second base 302 is placed with a given shape at the position to connect the adjacent through holes. The plane shape of the through hole is circular. The opening 32 extends to the position where the through hole is formed. The adjacent space structures 33 are thereby connected at the bottom.

In the shape shown in the perspective view of Fig. 19, a plurality of space structures 33 are formed on one base. The space structures 33 are arranged vertically and horizontally on the base surface. A groove-shaped opening 32 is formed between the space structures 33. On the surface of the plate base 30, the horizontal direction in Fig. 4 is a width direction, and the direction orthogonal to the width direction is a depth direction. This embodiment shows the plate base 30 having four space structures 33 in the width direction and four space structures 33 in the depth direction. Thus, sixteen space structures 33 are formed in matrix on the plate base 30. In this embodiment, the space structures 33 that are adjacent in the oblique direction are also connected through the opening 32. Specifically, the opening 32 is formed not only in the width and depth directions but also in the oblique direction therebetween. Further, in this embodiment, the openings 32 in the oblique direction do not cross each other unlike the embodiment shown in Fig. 18. Thus, the openings 32 in the oblique direction are all placed in the same direction. Therefore, one space structure 33 is connected with maximum six space structures 33 which are placed in its vicinity. The six space structures 33 may be arranged in a hexagonal shape.

As a method of forming pits and projections with a height of 1 µm or smaller, sandblasting may be used, for example. When forming smaller pits and projections, etching with Ar plasma or the like may be used.
As a method of forming pits and projections with a height of 1 µm or larger, dry etching, wet etching or the like of a silicon material or a glass material may be used, for example. A molding method of a resin material may be extrusion molding, injection molding, hot embossing, nanoimprinting, blowing, calendaring, casting, pressing or the like.

It is expected to control the extension direction of cultured cells with the use of a cell culture chamber having a plurality of pits or projections and a surface connected with the upper portion of the pits or the bottom portion of the projections.
If cell culture is performed with the use of a plate base or the like having a plurality of pits, it is possible to culture cells only in the upper portion connected in the pits. If the area of the bottom portion of the pits is too large, cultured cells can extend to the bottom portion also. Therefore, both or either one of the vertical and horizontal dimensions of the bottom portion of the pits is preferably in the range of 1 µm to 500 µm. If the vertical dimension of the bottom portion of the pits is set to the range of 1 µm to 500 µm and the horizontal dimension is set to 1 mm, 10 mm, or 50 mm, for example, in the culture of nerve cells or vascular endothelial cells, it is possible to obtain the length of cultured cells which is appropriate for the purpose.

If cell culture is performed with the use of a plate base or the like having a plurality of projections, it is expected to culture cells only in the bottom portion connected in the projections. If the area of the upper portion of the projections is too large, cultured cells can extend to the upper portion also. Therefore, both or either one of the vertical and horizontal dimensions of the upper portion of the projections is preferably in the range of 1 µm to 500 µm, If the vertical dimension of the upper portion of the projections is set to the range of 1 µm to 500 µm and the horizontal dimension is set to 1 mm, 10 mm, or 50 mm, for example, in the culture of nerve cells or vascular endothelial cells, it is possible to obtain the length of cultured cells which is appropriate for the purpose.

The depth or the height of the pits or projections is required for cells to recognize the pits or projections, and they are preferably in the range of 1 µm to 500 µm.

A desmosome that serves as pseudo-scaffolding in the cell culture is formed on a connecting surface or a side wall surface of the pits and projections, so that cells can grow on the connecting surface to be extendable, thereby enabling the control of the extension direction of the cells.
Although the fine pits and projections are formed on a plate base which is a separate unit from a chamber body in the above-described embodiments, it is not limited thereto, and the fine pits and projections may be formed at the bottom portion of the chamber body. Besides the cell culture chamber 100 which includes the plate base 30 having the fine pits and projections, a cell culture chamber in which the chamber body 40 is integrated with the plate base 30 and has the fine pits and projections formed on its bottom portion may be used. Because the chamber body and the fine pits and projections are integrated together, this eliminates the step of attaching the plate base 30 in the process of molding the cell culture chamber, thereby preventing the entrance of contamination.
Further, a flow channel for introducing fluid such as a culture solution or reagent may be placed in close proximity to the fine pits and projections.

### Industrial Applicability

The present invention is applicable to a cell culture chamber to culture cells that are isolated from tissue for use as assessment or examination, for example.

## Claims

1. A cell culture chamber at least comprising:
a chamber body to contain a culture;
a cover to cover an opening of the chamber body; and
a plate base to cover a through hole at a bottom portion of the chamber body,
wherein the plate base has fine pits and projections for cultivating the culture.

2. A cell culture chamber comprising:
a chamber body to contain a culture; and
a cover to cover an opening of the chamber body,
wherein a bottom portion of the chamber body has fine pits and projections for cultivating the culture.

3. The cell culture chamber according to claim 1, wherein a thickness of the plate base is smaller than a thickness of the bottom portion of the chamber body.

4. The cell culture chamber according to claim 3, wherein the thickness of the plate base is 0.03 mm to 1 mm.

5. The cell culture chamber according to one of claims 1 to 3, wherein a plurality of space structures are formed by the fine pits and projections, each space structure having a width of 0.01 µm to 1000 µm, a length of 0.01 µm to 1000 µm, and a height of 0.01 µm to 1000 µm.

6. The cell culture chamber according to one of claims 1 to 3, wherein a plurality of space structures are formed by the fine pits and projections, each space structure being connected with at least one adjacent space structure.

7. The cell culture chamber according to one of claims 1 to 3, wherein surface treatment is performed in a region having the fine pits and projections.

8. The cell culture chamber according to claim 1, wherein the plate base is a transparent resin base.

9. The cell culture chamber according to claim 1, wherein an area of the plate base is 20% to 60% of an area of the bottom portion of the chamber body.

10. The cell culture chamber according to claim 1, wherein
the bottom portion of the chamber body has a recessed portion in a region including the through hole, and
the plate base is inserted into the recessed portion.

11. The cell culture chamber according to claim 10, wherein the plate base is detachably fixed to the recessed portion by a fitting member.

12. The cell culture chamber according to claim 11, wherein the fitting member is made of a self-adhesive resin.

13. The cell culture chamber according to claim 11, wherein a thickness of the fitting member is equal to or larger than a depth of the recessed portion.

14. The cell culture chamber according to claim 1, wherein the through hole at the bottom portion is circular or rectangular.

15. The cell culture chamber according to claim 1, wherein a shape of the plate base is circular or rectangular.

16. The cell culture chamber according to claim 1 or 2, wherein the cover or the chamber body has an opening for perfusion.
